# EUROPEAN PATENT APPLICATION

(11) **EP 2 786 980 A1**
(43) Date of publication of application: **08.10.2014**
(21) Application number: 13198607.7
(22) Date of filing: 29.07.2008
(51) Int. Cl.: C07C 51/50, C07C 51/38, C07C 67/62, C08F 2/40

(54) **Method of decarboxylating maleic acid to acrylic acid**

(30) Priority: 31.08.2007 US 969215 P
(62) Divisional of application: 08782493.4
(71) Applicant: Arkema Inc., Philadelphia, PA 19103-3222 (US)
(72) Inventor: Fruchey, Olan, S., Hurricane, WV 25526 (US); Reeves, Christopher, T., Durham, NC 27712 (US); Brooks, William, C., Saint Albans, WV 25177 (US); Yang, Edmund, J., Houston, TX 77062 (US); Roundy, Roger, L., Hurricane, WV 25526 (US)
(74) Representative: Bailey, Sam Rogerson

(57) **Abstract**

A method of decarboxylating maleic acid to acrylic acid, the method comprising the step of contacting a solution comprising maleic acid with at least 500 ppm of an N-oxyl compound at a temperature of at least 120°C. The preferred N-oxyl compound is 4-hydroxy-TEMPO, in some cases in combination with at least one of Mn(II) and Cu(II).

## Description

### REFERENCE TO RELATED APPLICATION

This application claims the benefit of USSN 60/969,215, filed on August 31, 2007; which application is fully incorporated herein by reference.

### FIELD OF THE INVENTION

This invention relates to acrylic acid and acrylate processes. In one aspect, the invention relates to inhibiting unwanted acrylic and acrylate polymerization and the resulting fouling of process equipment while in another aspect, the invention relates to the use in the acrylic and acrylate processes of an inhibitor comprising a N-oxyl compound and a manganese ion. In still another aspect, the invention relates to an inhibitor comprising a high ratio of N-oxyl compound to manganese ion.

### BACKGROUND OF THE INVENTION

In the vapor phase manufacture of acrylic acid from propylene and air, the gaseous product stream is quenched with cold process liquids before it is subjected to purification. The quench produces a liquid stream of at least 25 weight percent (wt%) liquid acrylic acid, at most 75 wt% water, and minor amounts of various liquid by-products. The majority of the cold process liquids typically comprise aqueous acrylic acid taken from the base of the quench tower and pumped to the top of the tower. Since liquid acrylic acid is much more susceptible to unwanted vinyl polymerization than is gaseous acrylic acid, a polymerization inhibitor is typically added to the stream in this pump-around loop.

One family of commonly used inhibitors comprises hydroquinone either alone or in combination with a metal ion such as an ion of manganese or copper. While these inhibitors are effective, they can also result in the fouling of the process equipment, typically the purification equipment such as the steam generators, if the reaction water is recycled to the reactors. For example, hydroquinone will react with formaldehyde, a by-product of the acrylic acid process, to form a novolak-type polymer that will attach to equipment sidewalls and valves. This polymeric foulant then interferes with the heat exchange across the equipment sidewalls and the operation of the valves. This, in turn, can require frequent cleaning and down-time of the equipment.

This novolak-type polymer fouling can be eliminated by replacing the hydroquinone inhibitor with another water-soluble inhibitor that will not react with formaldehyde. For example, USP 5,504,243 describes a method for inhibiting the polymerization of (meth)acrylic acid and esters using an inhibitor comprising an N-oxyl compound, e.g., 4-hydroxy TEMPO (4-HT), in combination with two or more of a manganese salt, copper salt, 2,2,6,6-tetramethylpiperidine and a nitroso compound. The European equivalent of this patent, i.e., EP 0 685 447, describes a similar inhibitor, but this one comprising an N-oxyl compound in combination with one or more of a manganese salt, copper salt, 2,2,6,6-tetramethylpiperidine and a nitroso compound. However, in this equivalent the combination of 4-HT and a manganese salt provided only 4 hours of inhibition at 100°C when the 4-HT and manganese were present in a weight ratio between 1.33:1 to 1:1. Moreover, only a very small amount of inhibitor was used (about 10 parts per million (ppm) of each component), and typically at least 100 ppm of total inhibitor is required in a commercial distillation lower for good distribution of the inhibitor over the tower trays. Still further, the relatively large amount of manganese ion (metal) becomes an unwanted contributor to the ash from the incinerators in which the process waste stream (the ultimate repository for the inhibitor) is disposed. Yet further, this reference is void of any teaching that oxygen is an essential component of the inhibitor package.

### SUMMARY OF THE INVENTION

In one embodiment, the invention is a method of inhibiting the polymerization of a mono-ethylenically unsaturated carboxylic acid, ester or neutralized or partially neutralized salt, preferably a (meth)acrylic acid or ester, in an aqueous solution, the method comprising the step of mixing with the aqueous solution an inhibitor comprising (i) at least 50 ppm of an N-oxyl compound, and (ii) a manganese ion, the N-oxyl compound and manganese ion present in a N-oxyl compound to manganese ion weight ratio of 50:1 to less than 100:1 based on the mono-ethylenically unsaturated carboxylic acid, anhydride, ester or salt, in the presence of oxygen, typically fed as air. The amount of air fed to the tower is such that oxygen comprises at least 0.1 mole percent (mol%) of the gas within the tower.

In another embodiment, the invention is an inhibitor for stabilizing an aqueous solution of a mono-ethylenically unsaturated carboxylic acid, ester or neutralized or partially neutralized salt against polymerization, the inhibitor comprising an N-oxyl compound and a manganese ion at a weight ratio of 50:1 to less than 100:1 in the presence of oxygen.

In another embodiment, the invention is a stabilized aqueous solution of a mono-ethylenically unsaturated carboxylic acid, ester or neutralized or partially neutralized salt, the solution comprising (i) a mono-ethylenically unsaturated carboxylic acid, anhydride, ester or neutralized or partially neutralized salt, (ii) water, and (iii) an inhibitor comprising an N-oxyl compound and a manganese ion at a weight ratio of 50:1 to less than 100:1 in the presence of oxygen.

In yet another embodiment, a molecule of maleic acid, a by-product of the production of acrylic acid by the oxidation of propene, is decomposed (or decarboxylated) to one molecule of acrylic acid at an elevated temperature and in the presence of, i.e., catalyzed by, 4-hydroxy-TEMPO. This decomposition of maleic acid can typically occur in either the reboiler or dimer cracker of an acrylic acid equipment train, and at a temperature typically of at least 120C.

### DESCRIPTION OF THE PREFERRED EMBODIMENT

The numerical ranges in this disclosure include all values from and including the lower and the upper values, in increments of one unit, provided that there is a separation of at least two units between any lower value and any higher value. As an example, if a compositional, physical or other property, such as, for example, molecular weight, viscosity, melt index, etc., is from 100 to 1,000, it is intended that al individual values, such as 100, 101, 102, etc., and sub ranges, such as 100 to 144, 155 to 170, 197 to 200, etc., are expressly enumerated. For ranges containing values which are less than one or containing fractional numbers greater than one (e.g., 1.1, 1.5, etc.), one unit is considered to be 0.0001, 0.001, 0.01 or 0.1, as appropriate. For ranges containing single digit numbers less than ten (e.g., 1 to 5), one unit is typically considered to be 0.1. These are only examples of what is specifically intended, and all possible combinations of numerical values between the lowest value and the highest value enumerated, are to be considered to be expressly stated in this disclosure. Numerical ranges are provided within this disclosure for, among other things, the ratio of N-oxyl compound to manganese ion, the amount of (meth)acrylic acid or ester in solution, and various process parameters, e.g., temperature, pressure and the like.

"Polymer" means a polymeric compound prepared by polymerizing monomers, whether of the same or a different type. The generic term polymer thus embraces the term homopolymer, usually employed to refer to polymers prepared from only one type of monomer, and the terms copolymer and interpolymer as defined below.

"Copolymer", "interpolymer" and like terms means a polymer prepared by the polymerization of at least two different types of monomers. These generic terms include the traditional definition of copolymers, i.e., polymers prepared from two different types of monomers, and the more expansive definition of copolymers, i.e., polymers prepared from more than two different types of monomers, e.g., terpolymers, tetrapolymers, etc.

The prefix "(meth)" with generic terms, such as, for example, "acrylic acid" or "acrylate" broadens the base or root terms to include both acrylic and methacrylic, and acrylate and methacrylate species. Thus, the term "(meth)acrylic acid" includes acrylic acid and methacrylic acid, and the term "(meth)acrylate" includes acrylate and methacrylate species.

"Polymerization" and like terms means a chemical reaction in which a large number of relatively simple molecules combine to form a chain-like macromolecule, i.e., a polymer. The combining units are known as monomers.

"Inhibitor", "polymerization inhibitor", "stabilizer", "polymerization stabilizer" and like terms means a substance that will prevent or retard the polymerization of vinyl monomers under conditions of which the monomers would otherwise polymerize. The Michael addition of one acrylic acid or ester molecule to another is not a polymerization of acrylic acid or ester molecules.

"Solution" and like terms means a uniformly dispersed mixture at the molecular or ionic level of one or more substances (the solute) in one or more other substances (the solvent). In the context of an aqueous solution of acrylic acid or ester, the acrylic acid is the solute and water is the solvent, and the water can be present in amounts up to and exceeding 75 weight percent based on the weight of the solution. As used in this disclosure, solution also includes aqueous compositions in which water is present in only trace amounts, e.g., less than 0.01 weight percent based on the weight of the solution.

Although the invention is described in the context of (meth)acrylic acid and esters, it has applicability to other mono-ethylenically unsaturated carboxylic acids and their anhydrides, esters, and neutralized and partially neutralized salts. Examples of the alpha, beta (α,β)-mono-unsaturated carboxylic acids and esters that are stabilized against polymerization by this invention include acrylic acid, methacrylic acid, maleic acid, maleic anhydride, itaconic acid, ethacrylic acid, alpha-chloroacrylic acid, alpha-cyano acrylic acid, beta-methyl-acrylic acid (crotonic acid), alpha-phenyl acrylic acid, beta-acryloyloxy propionic acid, angelic acid, cinnamic acid, p-chloro cinnamic acid, citraconic acid, mesaconic acid, glutaconic acid, fumaric acid, maleic acid anhydride, itaconic anhydride, half esters or half amides of maleic, fumaric and itaconic acid, crotonic acid, acrylamide, methacrylamide and their N and N, N dialkyl derivatives containing 1-18 carbon alkyl groups, alkyl acrylates and methacrylates containing 1-18 carbon alkyl groups (e.g., methyl, ethyl, propyl acrylate and methacrylate), and the like.

The family of free radical polymerization inhibitors used in the practice of this invention is based on 4-hydroxy-2,2,6,6-tetramethylpiperidin-1-oxyl also known as nitroxyl 2, or NR 1, or 4-oxypiperidol, or tanol, or tempol, or tmpn, or probably most commonly, 4-hydroxy-TEMPO, or h-TEMPO or even more simply, 4-HT. These TEMPO compounds are also known as N-oxyl or, more simply, oxyl compounds or stabilizers, or HARTs (hindered amine radical traps), or HALS (hindered amine light stabilizers). The TEMPO family members are differentiated by various groups located at the 4 position of the ring. The most commonly known member of the family is 4-hydroxy TEMPO (4-HT), a preferred N-oxyl compound for use in this invention, in which a hydroxyl group is located at the 4 position of the ring (see formula (I)): 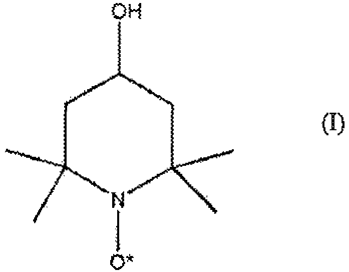 The TEMPO compounds from which a derivative, particularly the ether, ester and urethane derivates, can be prepared are of formula (II):

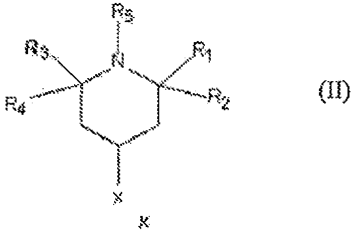

The ether, ester and urethane derivatives of a TEMPO compound that can be used as a component of the polymerization inhibitors in the practice of this invention have the chemical structural formula of (III): 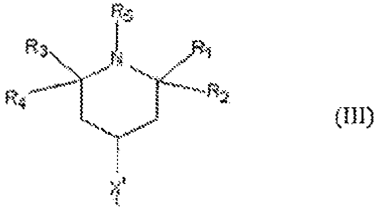 in which
X of formula II is any group that can react with another compound, e.g., an alcohol, a carboxylic acid, an alkyl sulfate, an isocyanate, etc., to form the ether, ester or urethane group (or corresponding sulfur, phosphorus or amine derivative) of formula III, and preferably X is hydroxyl, amine, mercaptan, phosphino (H₂P-), phosphinyl (H₂P(O)-) or silyl (H₃Si-) group, and more preferably X is hydroxyl;
X' of formula III is at least a divalent atom, preferably an atom of oxygen, sulfur, nitrogen, phosphorus or silicon, more preferably an atom of oxygen or sulfur and most preferably an atom of oxygen;
and with respect to both formulae II and III
R₁-R₄ are each independently a C₁-₁₂ hydrocarbyl group, or any of the R₁-R₄ groups can join with one or more of the other R₁-R₄ groups to form one or more hydrocarbyl rings, preferably with at least a 5 carbon atoms;
R₅ is an oxyl (O•) radical;
R₆ is a hydrogen or C₁₋₁₂ hydrocarbyl or carboxyl group, or a urethane group of the formula

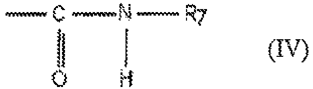

With the proviso that if the R₁-R₄ groups are methyl, then R₆ is not hydrogen;
and
R₇ is a C₂₋₃₀ hydrocarbyl group.

As here used, "ether, ester and urethane derivatives" are the compounds of formula III in which X' is a divalent oxygen radical. The hydrocarbyl groups of R₁-R₇ include, but are not limited to, alkyl, aryl, aralkyl, cycloalkyl, alkenyl, and the like. Preferably, R₁-R₄ are each independently a C₁₋₄ alkyl group and more preferably, R₁-R₄ are each independently methyl groups. Preferably R₆ is a C₁₋₁₂ alkyl, or a C₁₋₁₂ alkyl carboxyl or an aryl carboxyl group, or a urethane group, and more preferably a C₁₋₈ alkyl group, or benzoic acid group, or a urethane group. Preferably R₇ is a C₅₋₃₀, alkyl group, more preferably a C₅₋₂₀ alkyl group. Representative ether and urethane derivatives of 4-hydroxy-TEMPO include methyl ether TEMPO, butyl ether TEMPO, hexyl ether TEMPO, allyl ether TEMPO and stearyl urethane TEMPO.

As practiced in this invention, 4-HT and Mn(II) are added to streams containing acrylic acid. Under the conditions of a distillation system, the 4-HT reacts with acrylic acid forming either the 4-HT acrylate ester (V below, i.e., the ester formed from the alcohol function of 4-HT with the carboxylic acid function of acrylic acid) or the Michael adduct Beta-(4-oxy TEMPO) propionic acid (VI below, i.e., the Michael adduct formed when the alcohol function of 4-HT adds across the double bond of the acrylic acid). These are the two major products which are formed, and in each case the derivatives still have the active nitroxyl radical present. This means they are still potent inhibitors.

Furthermore, the 4-HT can react to a lesser extent (due to the lower concentration of these species being present in the mixture) with acetic acid forming the 4-HT acetate ester (VII below) and with the acrylic acid dimer forming the corresponding ester (VIII below) and Michael adducts (IX below). Thus for all practical purposes, the true inhibitor system in acrylic acid distillation is these 4-HT derivatives in conjunction with the Mn(II) ion. Although gas chromatography (GC) and high pressure liquid chromotography (HPLC) analysis of the distillation streams will not show the presence of 4-HT, these and other potential derivatives can be found. This means that the inhibitor package (i.e., the active nitroxyl derivative in conjunction with manganese ion) can be formed either *in situ* by the direct addition of 4-HT and manganese ion to the process, or it can be preformed by external reaction with acrylic acid and then added to the process along with manganese ion.

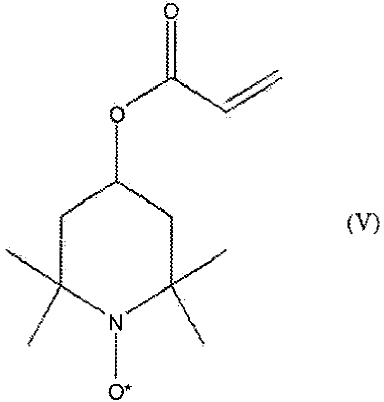

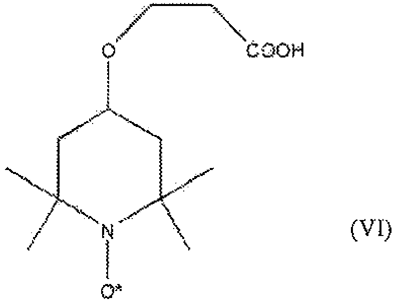

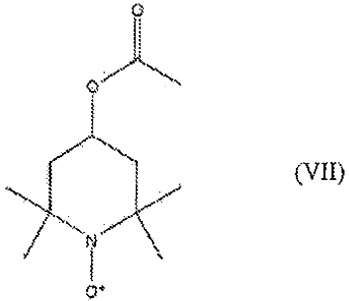

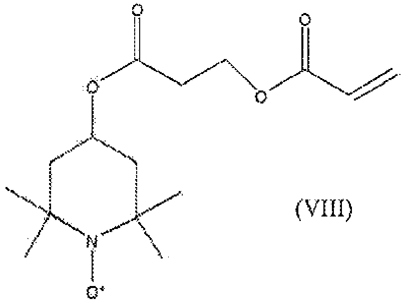

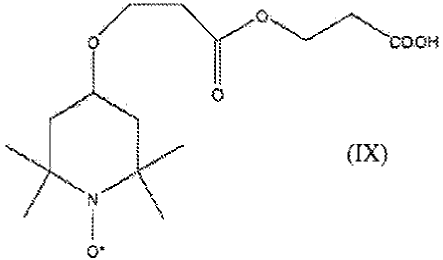

The manganese ion used in the practice of this invention is preferably of a +2 valence, and it is typically derived from a manganese salt such as, for example, manganese dialkyldithiocarbamate (the alkyl groups are selected from methyl, ethyl, propyl and butyl and may be same or different with each other), manganese diphenyldithiocarbamate, manganese formate, manganese acetate, manganese octanate, manganese naphthenate, manganese ethylenediaminetetraacetate, and the like. One or more kinds of them may be used.

The N-oxyl compound to manganese ion weight ratio is 50:1 to less than 100:1, preferably to less than 75:1 and more preferably to less than 60:1, based on the aqueous solution of (meth)acrylic acid or ester and water. At these ratios and with a minimum of at least 50 ppm 4-HT, a super stabilizing effect is imparted to an aqueous solution of (meth)acrylic acid, i.e., the majority (e.g., >50%) of the (meth)acrylic acid will dimerize *via* Michael addition before vinyl polymerization can occur. If liquid acrylic acid is held at an elevated temperature (e.g., 113°C for 72 hours), most of the acrylic acid is converted to a dimer and very little (e.g., <10%) free acrylic acid remains. Neither N-oxyl compound nor manganese ion alone produces this result, and the use of more manganese ion, e.g., the N-oxyl compound to manganese ion ratio of 1:1 taught in the art, adds to metal content of the waste streams that are ultimately incinerated. The incineration produces an ash with a metal content which requires disposal in an environmentally, and usually costly, manner.

The components of the inhibitor i.e., the N-oxyl compound and the manganese ion precursor (e.g., a salt), can either be pre-mixed or added independently to the aqueous solution of (meth)acrylic acid or ester. The inhibitor (N-oxyl compound plus manganese ion precursor) are added either to the water used to make the aqueous solution of (meth)acrylic acid or ester, or to the solution itself, in amount of at least 50, preferably at least 100 and more preferably at least 200, ppm. In the context of a gas-phase process for the manufacture of (meth)acrylic acid or ester, the inhibitor is usually added at the quench stage of the process. If the inhibitor is pre-mixed, then it is typically added to the cold process liquids before or at the time that these liquids enter the top of the tower in which the gaseous (meth)acrylic acid or ester is quenched. The temperature of these process liquids is typically between 15 and 30°C. If the components of the inhibitor are added to the process independent of one another, then they are typically added to the quench liquids before the liquids enter the quench tower, and additional amounts of the components can be added to different sections of the quench tower to insure a synergistic interaction of components throughout the purification process.

An inhibitor solution is easily prepared by adding manganese acetate solids or solution to a commercially available ten percent aqueous solution of 4-hydroxy-TEMPO. In a preferred embodiment, this solution is then fed directly to the pump-around loop of the absorber (quench) tower or fed to a distillation tower by way of the reflux, i.e., that pan of the condensed overhead vapor which is returned to the top tray. The inhibitor solution can also be added to a tower condenser, e.g., a quench condenser, and/or to a simple flasher, i.e., a one-stage distillation unit.

In one embodiment of this invention, the inhibitor of N-oxyl compound and manganese ion is used in combination with an inhibitor that is soluble in an organic medium. The N-oxyl compound, particularly 4-hydorxy TEMPO, and the manganese salt are both very water-soluble and as such, only partially partition into the organic phase within the extraction and/or distillation towers. This, in turn, only provides partial stabilization against vinyl polymerization of any (meth)acrylic acid or ester that is contained with the organic phase. To protect against vinyl polymerization in the organic phase, the inhibitor can comprise one or more additional components that provides this function, e.g., phenothiazine. This additional component or blend of components, if present, is usually present in an amount of 50, preferably 100 and more preferably 200, ppm.

4-Hydroxy-TEMPO by itself is stoichiometrically consumed as an inhibitor, and it does not require the presence of oxygen to function as an inhibitor. The presence of Mn(II) and oxygen allows for the regeneration of those 4-hydroxy-TEMPOs that have trapped a radical, thus making it a catalytic inhibitor. The oxygen is provided to the towers by air injection into either the reboiler or base of the tower. The Mn(II) serves two purposes. One purpose is to act as an oxidation catalyst for regeneration of 4-hydroxy-TEMPOs that have trapped a radical. Another purpose is when oxidized to Mn(III), it can act as an inhibitor *via* one electron transfer from a carbon centered radical forming a carbo-cation and Mn(II), and thus preventing polymerization.

At the proper levels and ratios, 4-HT in conjunction with Mn(II) can provide super stabilization of acrylic acid at 113°C. The preferred inhibitor ratios for acrylic acid distillation of this synergistic inhibitor mix is 100/1 (4-HT/Mn(II)) with a more preferred ratio of 50/1. In a distillation tower the inhibitor concentration on each tray should be at least 50 ppm 4-HT/1 ppm Mn(II), the more preferred level is at least 100 ppm 4-HT/2 ppm M-n(II) and an even more preferred concentration is at least 200 ppm 4-HT/4 ppm Mn(II). At lower levels inhibitor distribution on the trays can be a concern and lead to fouling due to poor distribution in commercial scale equipment. Higher ratios of Mn(II) in the inhibitor mix will provide inhibition but lead to disposal issues. In other words at a 1/1 ratio [4-HT/Mn(II)] and a minimum of 50 ppm 4-HT, the amount of ash generated in an incinerator used to dispose of process heavy ends would present an environmental problem. This is not an issue at the 50/1 ratio.

One surprising advantage of 4-HT in acrylic acid purification is its ability to catalyze the decomposition of maleic acid in a finishing column base section and/or reboiler and even more effectively, in a dimer cracker. The 4-HT acts as a catalyst for the decomposition of maleic acid *via* decarboxylation yielding acrylic acid. The high temperature (typically above 150°C) of the dimer cracker is preferred for this reaction although this reaction proceeds well at temperatures as low as 120°C. This characteristic of 4-HT is not observed with other non-TEMPO acrylic acid inhibitors, e.g., PTZ or HQ. This means that 4-HT not only prevents fouling in the purification system but also converts a reactor by-product into product in the purification system thus maximizing yield. The typical concentration of 4-HT in either the finishing column reboiler or dimer cracker is at least 500, preferably at least 1,000 and more preferably at least 2,000, ppm. Because 4-HT is a high boiler, it tends to concentrate in the reboiler of a finishing tower which can be equipped with a dimmer cracker. The typical temperature for cracking the dimer in the presence of 4-HT is at least 150, preferably at least 170 and more preferably at least 200, °C.

The to following examples further illustrate the invention. Unless otherwise noted, all parts and percentages are by weight.

### SPECIFIC EMBODIMENTS

### Experimental Procedure

### Standard Induction Time Measurement:

Ten milliliter samples of purified acrylic acid (containing known amounts of inhibitor) are placed in DOT (Department of Transportation) tubes. DOT tubes are 10 mL glass ampoules equipped with a 6 inch long, ½ inch diameter glass tubing neck, which are fitted with % inch Swagelok^{tm} nylon caps. The caps are used to support the tubes which extend through twelve 3/8 inch holes in a 6 inch diameter circular plastic block that is about one inch thick. The block is attached by an offset center support rod to an overhead stirrer. The bulb portion of the tubes are then submerged in a constant temperature silicon oil bath held at 113°C for 72 hours while being rotated (*via* the overhead stirrer) at about 50 rpm. The tubes are visually monitored during this time for signs of polymer formation (i.e. cloudiness, presence of solids or increased viscosity). The amount of time until first signs of polymer formation is defined as the induction time (or on-set time). Each run consists of six replicate DOT tubes, and the induction time is the average of these six replicates.

### Fischer & Porter Pressure Tube Test;

Two glass Fischer & Porter pressure tubes (80 mL volume) are charged with 50 mL of the following aqueous solutions:
a) 10% acetic acid / 5% acrylic acid / 1000 ppm HQ / 10 ppm Mn(II) / balance water
b) 10% acetic acid / 5% acrylic acid / 500 ppm 4-HT / 10 ppm Mn(II) / balance water.

The pressure tubes are scaled with pressure heads which are equipped with a pressure gauge. After securing the heads, the tubes are submerged (covering only the liquid level of the tubes) in a constant temperature oil bath at 149°C for 4 hrs with an autogenic pressure of 25 psig. The tubes are visually monitored during this time for the presence of polymer.

### Purification of Acrylic Acid via Melt Crystallization;

Three liters of Aldrich glacial acrylic acid (inhibited with 200 ppm MeHQ, monomethyl ether of hydroquinone) is placed in a plastic beaker and then allowed to freeze in the refrigerator at 6°C overnight. The next day the frozen acrylic acid is removed from the refrigerator and a center core containing about one quarter of the volume of the material is cut out and removed from the heaker. The contents left in the beaker are warmed in a 20°C water bath to melt the frozen material. After melting the contents are swirled to evenly mix the remaining inhibitor and then again placed in the refrigerator for a second crystallization. This is repeated for a total of three crystallizations, yielding about 1 kg of purified acrylic acid which contained about 50 ppm of MeHQ inhibitor. This material is used for part of the induction time studies.

### Purification of Acrylic Acid via Rotary Evaporator:

A one liter, round-bottom flask is charged with about 400 mL of Aldrich glacial acrylic acid (inhibited with 200 ppm MeHQ), The flask is attached to a rotary evaporator equipped with a 50°C water bath and vacuum pulled (about 20 mm Hg absolute pressure). About 250 mL of distilled acrylic acid is collected in the ice-water cooled receiver. This is melted and then subjected to rotary evaporation again to produce material that contains less than 1 ppm MeHQ and is used for the rest of the induction time studies.

### Chemicals Employed:

The following materials, all from Aldrich Chemical Co., are used in these examples.
Acrylic Acid (99%)
Copper (II) Acetate (97%)
Hydroquinone (HQ)(99%)
Manganese (II) Acetate (tetrahydrate)
Phenothiazine (PTZ)(>98%)
4-Hydroxy TEMPO (4-HT)(solid)

### Results and Discussion

### Standard Induction Time Test Results:

4-HT is tested to determine its efficacy as a polymerization inhibitor for acrylic acid. Potential adverse inhibition interactions of 4-HT with the other inhibitors are also evaluated. The results from the standard lab induction time tests are contained in Table I. All tests are run at 113°C, the maximum reboiler wall temperature expected in a solvent removal tower. Also all tests are conducted under an air headspace.

**TABLE I**

| Inhibitor Induction Times | | | | | | | |
|---|---|---|---|---|---|---|---|
| 4-HT (ppm) | HQ (ppm) | PTZ (ppm) | MeHQ (ppm) | Mn(II)^{d} (ppm) | Cu(II)^{e} (ppm) | Temp (°C) | Induction Time (hrs) |
| 50 | 0 | 0 | 0 | 0 | 0 | 113 | 30-46^{b} |
| 50 | 0 | 0 | 0 | 1 | 0 | 113 | >72^{a} |
| 50 | 0 | 0 | 50 | 0 | 0 | 113 | 30-46^{b} |
| 50 | 0 | 0 | 50 | 1 | 0 | 113 | >72^{a} |
| 50 | 0 | 0 | 0 | 1 | 1 | 113 | >72^{a} |
| 50 | 0 | 0 | 50 | 1 | 1 | 113 | >72^{a} |
| 50 | 0 | 0 | 50 | 0 | 1 | 113 | 30 |
| 50 | 100 | 0 | 50 | 10 | 0 | 113 | >72^{a} |
| 50 | 100 | 100 | 50 | 1 | 0 | 113 | >72^{a} |
| 100 | 0 | 0 | 50 | 0 | 0 | 113 | 54 |
| 0 | 100 | 0 | 50 | 0 | 0 | 113 | 1.5 |
| 0 | 0 | 100 | 50 | 0 | 0 | 113 | 30-46^{b} |
| 100 | 0 | 100 | 50 | 0 | 0 | 113 | 51 |
| 100 | 100 | 0 | 50 | 0 | 0 | 113 | 55-70^{e} |
| 100 | 0 | 0 | 50 | 10 | 0 | 113 | >72^{a} |
| 100 | 100 | 1000 | 50 | 0 | 0 | 113 | 55-70^{c} |
| 0 | 100 | 0 | 50 | 10 | 0 | 113 | >72^{a} |
| 3000 | 3000 | 0 | 50 | 0 | 0 | 113 | >72^{a} |
| 0 | 0 | 0 | 0 | 1 | 0 | 113 | 4.5 |

| | | | | | | | |
|---|---|---|---|---|---|---|---|
| a) Super stabilized, >90% of acrylic acid had undergone Michael additions b) All samples polymerized overnight between 30 and 46 hours c) All samples polymerized overnight between 55 and 70 hours d) Mn(II) ion concentration in solution (was added as manganese acetate) e) Cu(II) ion concentration in solution (was added as cupric acetate) | | | | | | | |

Induction times are directly related to inhibitor efficacy (i.e. a longer induction time is observed for better inhibitors). The data in Table I clearly show that on a per pound basis, 4-HT is significantly better than HQ. In fact, it is at least an order of magnitude better than HQ. Also, 4-HT appears to be better than PTZ (a commonly used acrylic acid process inhibitor). Surprisingly, a synergistic effect between 4-HT and Mn(II) in the presence of oxygen is demonstrated. This effect has not been reported in the open literature. An inhibitor package consisting of 50 ppm 4-HT and 1 ppm of Mn(II) in the presence of oxygen provides super stabilization, i.e., the acrylic acid contains sufficient inhibitor to allow most of the acrylic acid to undergo dimerization before vinyl polymerization occurs. In other words, the induction time exceeds the amount of time it take for >90% of the acrylic acid to undergo Michael additions (which means that after 72 hours at 113°C very little free acrylic acid is still present). However, the acrylic acid dimer can also polymerize to give a clear glassy solid if heating is continued for an extended period.

This synergistic effect between 4-HT and Mn(II) is similar to the synergistic effect that has been documented for HQ (or MeHQ) and Mn(II). Previous studies have shown that 100 ppm HQ and 1 ppm Mn(II) also provides super stabilization for acrylic acid. However, the inhibitor of this invention achieved super stabilization with half as much 4-HT as HQ at the 1 ppm Mn(II) level. This means that the 50 ppm 4-HT and 1 ppm Mn(II) inhibitor package can be substituted for a commercial HQ/Mn package with no loss of efficacy.

The data in Table I also demonstrate an absence of negative interactions, i.e., loss of inhibitor capability, between 4-HT and the preferred process co-inhibitor (PTZ) and as such, these can be mixed as desired. This can result in an improvement for those areas of the tower in which phasing may occur because of the increased solubility of 4-HT in water (I part 4-HT in 1 part water) compared to the less soluble HQ (1 part HQ in 14 parts water).

### Fischer & Porter Pressure Tube Test Results:

The Fischer & Porter pressure tube test is a simulation of conditions expected in steam generators. After fours hours at 149°C the synthetic reaction water (85% water / 10% acetic acid / 5% acrylic acid) shows no signs of vinyl polymerization for either the 4-HT/Mn(II) or HQ/Mn(II) inhibited solutions. This means that both inhibitor packages provide vinyl polymerization protection for times significantly greater than commercial steam generator residence times. In both cases the inhibitor concentration employed (1000 ppm HQ / 10 ppm Mn⁺² and 500 ppm 4-HT / 10 ppm Mn⁺²) are those expected (based on unit mass balance) if a 100 ppm HQ / 1 ppm Mn (II) inhibitor package is replared with a 50 ppm 4-HT / 1 ppm Mn (II) inhibitor package, This test simulates a steam generator residue and reflects the fact that the inhibitors would be concentrated in this stream.

### Decarboxylation of Maleic Acid

To demonstrate the ability of 4-HT to catalyze the decarboxylation of maleic acid into acrylic acid, a one percent solution of maleic acid in propionic acid was heated at 135C for four hours. The 4-HT, either by itself or in combination with Mn(II) or Cu(II) (Samples 1-3), successfully reduced the amount of maleic acid as compared to maleic acid in the absence of 4-HT (Sample 4).

**Table 2**

| Effect of 4-HT to Catalyze the Decarboxylation of Maleic Acid into Acrylic Acid | | | | | |
|---|---|---|---|---|---|
| **Sample** | **4-HT (ppm)** | **Mn(II) (ppm)** | **Cu(II) (ppm) @** | **% MA Time = 0 hr** | **%MA @ Time = 4 hr** |
| 1 | 2000 | 0 | 0 | 1.008 | 0.797 |
| 2 | 2000 | 0 | 700 | 0.962 | 0.677 |
| 3 | 2000 | 40 | 0 | 1.005 | 0.87 |
| 4 (Comp) | 0 | 0 | 0 | 0.986 | 0.985 |

Although the invention has been described in considerable detail, this detail is for the purpose of illustration and is not to be construed as a limitation on the scope of the invention as described in the pending claims. All U.S. patents and published patent applications identified above are incorporated herein by reference.

The following numbered clauses define aspects of the present invention and form part of the present disclosure.
1. A method of inhibiting the vinyl polymerization of a mono-ethylenically unsaturated carboxylic acid, ester or neutralized or partially neutralized salt in an aqueous solution and in the presence of oxygen, the method comprising the step of mixing with the aqueous solution an inhibitor comprising (i) at least 50 ppm of an N-oxyl compound, and (ii) a manganese ion, the N-oxyl compound and manganese ion present in a N-oxyl compound to manganese ion weight ratio of 50:1 to less than 100:1 based on the mono-ethylenically unsaturated carboxylic acid, anhydride, ester or salt.
2. The method of Clause 1 in which the mono-ethylenically unsaturated carboxylic acid, ester or neutralized or partially neutralized salt is a (meth)acrylic acid or ester.
3. The method of Clause 2 in which the N-oxyl compound is 4-hydroxy TEMPO.
4. The method of Clause 3 in which the manganese ion has a +2 valence.
5. The method of Clause 4 in which the aqueous solution is within a quench tower.
6. The method of Clause 4 in which the aqueous solution is within a distillation tower, the tower (i) comprising or in combination with at least one of a reboiler, base section, condenser and flasher, and (ii) containing a gas, and air is fed to the tower through the reboiler or base.
7. The method of Clause 6 in which sufficient air is fed to the tower such that the oxygen content within the tower is at least 0.1 mole percent of the gas within the tower.
8. The method of Clause 4 in which the (meth)acrylic acid or ester is acrylic acid or methacrylic acid.
9. The method of Clause 8 in which the inhibitor is present in the aqueous solution in an amount of at least 100 ppm.
10. The method of Clause 9 in which the N-oxyl compound and manganese ion are present in a N-oxyl compound to manganese ion weight ratio of 50:1 to less than 75:1.
11. The method of Clause 10 in which the inhibitor further comprises phenothiazine.
12. An inhibitor for stabilizing an aqueous solution of a mono-ethylenically unsaturated carboxylic acid, ester or neutralized or partially neutralized salt against polymerization in the presence of oxygen, the inhibitor comprising an N-oxyl compound and a manganese ion at a weight ratio of 50:1 to less than 100:1.
13. The inhibitor of Clause 12 in which the mono-ethylenically unsaturated carboxylic acid, ester or neutralized or partially neutralized salt is a (meth)acrylic acid or ester.
14. The inhibitor of Clause 13 in which the N-oxyl compound is 4-hydroxy TEMPO.
15. The inhibitor of Clause 14 in which the manganese ion has a +2 valence.
16. The inhibitor of Clause 15 in which the (meth)acrylic acid or ester is acrylic acid or methacrylic acid.
17. An aqueous solution of a mono-ethylenically unsaturated carboxylic acid, ester or neutralized or partially neutralized salt stabilized against polymerization in the presence of oxygen, the solution comprising (a) a mono-ethylenically unsaturated carboxylic acid, ester or neutralized or partially neutralized salt, (b) water, and (c) an inhibitor comprising (i) at least 50 ppm of an N-oxyl compound, and (ii) a manganese ion, the N-oxyl compound and manganese ion present in a N-oxyl compound to manganese ion weight ratio of 50:1 to less than 100:1 based on the mono-ethylenically unsaturated carboxylic acid, anhydride, ester or salt.
18. The solution of Clause 17 in which the mono-ethylenically unsaturated carboxylic acid, ester or neutralized or partially neutralized salt is a (meth)acrylic acid or ester.
19. The solution of Clause 18 in which the N-oxyl compound is 4-hydroxy TEMPO.
20. The solution of Clause 19 in which the manganese ion has a +2 valence.
21. The solution of Clause 20 in which the (meth)acrylic acid or ester is acrylic acid or methacrylic acid.
22. The solution of Clause 21 in which water is present in a trace amount.
23. In the method for inhibiting the vinyl polymerization of a mono-ethylenically unsaturated carboxylic acid, ester or neutralized or partially neutralized salt in an aqueous solution and in the presence of oxygen, the method comprising the step of mixing with the aqueous solution an inhibitor comprising an N-oxyl compound and manganese ion, the improvement comprising the step of mixing with the aqueous solution an inhibitor comprising (i) at least 50 ppm of an N-oxyl compound, and (ii) a manganese ion, the N-oxyl compound and manganese ion present in a N-oxyl compound to manganese ion weight ratio of 50:1 to less than 100:1 based on the mono-ethylenically unsaturated carboxylic acid, anhydride, ester or salt.
24. A method of decarboxylating maleic acid to acrylic acid, the method comprising the step of contacting a solution comprising maleic acid with at least 500 ppm of an N-oxyl compound at a temperature of at least 120°C.
25. The method of Clause 24 in which the N-oxyl compound is 4-hydroxy-TEMPO.
26. The method of Clause 25 in which the solution of maleic acid is contacted with the 4-hydroxy-TEMPO in combination with at least one of Mn(II) and Cu(II).
27. An inhibitor for stabilizing an aqueous solution of a mono-ethylenically unsaturated carboxylic acid, ester or neutralized or partially neutralized salt against polymerization in the presence of oxygen, the inhibitor comprising (i) a reaction product of an N-oxyl compound and at least one of (meth)acrylic acid, acetic acid and acrylic acid dimer, and (ii) a manganese ion at a reaction product to manganese ion weight ratio of 50:1 to less than 100:1.
28. The inhibitor of Clause 27 in which the N-oxyl compound is 4-hydroxy-TEMPO.
29. A method of inhibiting the vinyl polymerization of a mono-ethylenically unsaturated carboxylic acid, ester or neutralized or partially neutralized salt in an aqueous solution and in the presence of oxygen, the method comprising the steps of (a) preparing an inhibitor comprising (i) a reaction product of an N-oxyl compound and at least one of (meth)acrylic acid, acetic acid and acrylic acid dimer, and (ii) a manganese ion at a reaction product to manganese ion weight ratio of 50:1 to less than 100:1, and (b) mixing the inhibitor with the aqueous solution such that the inhibitor is present in an amount of at least 50 ppm based on the weight of the aqueous solution.

## Claims

1. A method of decarboxylating maleic acid to acrylic acid, the method comprising the step of contacting a solution comprising maleic acid with at least 500 ppm of an N-oxyl compound at a temperature of at least 120°C.

2. The method of Claim 1 in which the N-oxyl compound is 4-hydroxy-TEMPO.

3. The method of Claim 2 in which the solution of maleic acid is contacted with the 4-hydroxy-TEMPO in combination with at least one of Mn(II) and Cu(II).

4. The method of claim 1, in which the step of contacting a solution comprising maleic acid is performed in a finishing column base section and/or reboiler.

5. The method of claim 1, in which the step of contacting a solution comprising maleic acid is performed in a dimer cracker.

6. The method of claim 1, in which the step of contacting a solution comprising maleic acid is performed in the reboiler of a finishing tower equipped with a dimer cracker.

7. The method of claim 1, in which said N-oxyl compound comprises a reaction product of an N-oxyl compound.
